# EUROPEAN PATENT SPECIFICATION

(11) **EP 2 463 373 B1**
(45) Date of publication and mention of the grant of the patent: **12.04.2017**
(21) Application number: 10755073.3
(22) Date of filing: 21.07.2010
(51) Int. Cl.: C12N 15/29, C07K 14/415, C12N 15/82, A01H 1/00

(54) **GENE CODING FOR AN AUXIN RECEPTOR PROTEIN DERIVED FROM THE RICE PLANT, AND APPLICATIONS THEREFOR**
GENKODIERUNG FÜR EIN AUS DER REISPFLANZE ABGELEITETES AUXINREZEPTORPROTEIN UND ANWENDUNGEN DAVON
GÈNE CODANT POUR UNE PROTÉINE DE RÉCEPTEUR D'AUXINE DÉRIVÉE DE LA PLANTE DE RIZ, ET APPLICATIONS POUR CELUI-CI

(30) Priority: 04.08.2009 KR 20090071553
(43) Date of publication of application: 13.06.2012
(73) Proprietor: Republic of Korea (Management: Rural Development Administration), Suwon, Gyeonggi-do 441-707 (KR)
(72) Inventor: KIM, Dong Hern, Suwon-si Gyeonggi-Do 441-763 (KR); LEE, Keun Pyo, Suwon-si Gyeonggi-Do 441-440 (KR); KIM, Myong Il, Seoul 138-955 (KR); KWON, Yu Jin, Seoul 152-090 (KR); KIM, Yong Sam, Daejeon 305-720 (KR)
(74) Representative: Hiebl, Inge Elisabeth
(86) International application number: PCT/KR2010/004754
(87) International publication number: WO 2011/016632

(56) References cited:
- US-A1- 2006 123 505
- DATABASE UniProt [Online] 20 March 2007 (2007-03-20), "SubName: Full=Putative uncharacterized protein;", XP002689023, retrieved from EBI accession no. UNIPROT:A2YHP3 Database accession no. A2YHP3
- STEFFEN VANNESTE ET AL: "Auxin: A Trigger for Change in Plant Development", CELL, vol. 136, no. 6, 1 March 2009 (2009-03-01) , pages 1005-1016, XP055046979, ISSN: 0092-8674, DOI: 10.1016/j.cell.2009.03.001
- KEITHANNE MOCKAITIS ET AL: "Auxin Receptors and Plant Development: A New Signaling Paradigm", ANNUAL REVIEW OF CELL AND DEVELOPMENTAL BIOLOGY, vol. 24, no. 1, 1 November 2008 (2008-11-01), pages 55-80, XP055046978, ISSN: 1081-0706, DOI: 10.1146/annurev.cellbio.23.090506.123214
- DATABASE DATABASE NCBI ORYZA SATIVA JAPONICA GROUP 14 February 2008 'Os07g0121000', XP008158931 Database accession no. NP 001058783.1
- DATABASE DATABASE NCBI ORYZA SATIVA JAPONICA GROUP 14 February 2008 'Os07g0120900', XP008158932 Database accession no. NP_001058784.1
- DATABASE DATABASE NCBI ORYZA SATIVA JAPONICA GROUP 14 February 2008 'Os07g0121100', XP008158933 Database accession no. NP 001058785.1
- DATABASE DATABASE NCBI ORYZA SATIVA JAPONICA GROUP 14 February 2008 'Os07g0122000', XP008158934 Database accession no. NP 001058789.1
- DATABASE DATABASE NCBI ORYZA SATIVA JAPONICA GROUP 14 February 2008 'Os07g0122300', XP008158939 Database accession no. NP 001058792.1
- DATABASE DATABASE GENBANK ORYZA SATIVA JAPONICA GROUP 21 April 2008 'Auxin-binding protein', XP008158940 Database accession no. ACC59786.1
- 'Proceeding 33rd PGRSA 2006 Annual Meeting.', 2006 deel XIAO, L. ET AL.: 'Molecular cloning and analysis of oryza sativa auxin-binding protein 1 gene.', pages 123 - 130, XP008158941

## Description

### TECHNICAL FIELD

The present invention is related to a method of improving traits of a plant comprising transforming a plant with a recombinant plant expression vector, wherein the plant expression vector comprises a gene encoding an auxin receptor protein involved in the activation of a proton pump in the plasma membrane of a plant derived from rice, wherein the protein has an amino acid sequence of SEQ ID NO: 1 wherein the improved traits are increased plant growth or increased seed size and to plants, the use of a recombinant plant expression vector and a composition comprising a gene encoding SEQ ID NO: 1 for producing by transformation a plant having increased plant growth or seed having increased seed size. More specifically, the present invention discloses an auxin receptor protein ABP57 derived from crops like rice, corn, and etc., gene and analogous gene thereof, and its use for the improvement of traits of a plant that are related to the activity of a proton pump of a plant like growth promotion, etc.

### BACKGROUND ART

Auxin is a plant hormone which is involved in various traits of a plant. Hormone's activity of controlling physiology is achieved by a binding of a hormone to a receptor protein and a series of signal transduction pathways that are derived therefrom. Thus, characterization of a hormone receptor is the most basic requirement to understand the control mechanism of a hormone (Badescu and Napier (2006) Trends Plant Sci 11, 217-223**).** Studies to identify the receptor for auxin, which is one of the basic plant hormones, have been carried out for a long time, and as a result it was reported that the proteins like ABP1, TIR1, and ABP57, etc. are able to function as an auxin receptor (Hertel et al. (1972) Planta 107, 325-340**)**

The data base entry UniProt: A2YHP3 discloses a putative and uncharacterized protein from Oryza Sativa Subsp. Indica.

US 2006/0123505 discloses full-length cDNAs of the rice genome and their use for various purposes.

Steffen Vaneste et al: "Auxin: A Trigger for Change in Plant Development", Cell, vol. 136, no. 6, 2009-03-01, pages 1005-1016 discloses auxin-dependent TIR1 activity, which leads to ubiquitination-based degradation of transcriptional repressors and complex transcriptional reprogramming upon auxin binding.

Keithanne Mockaitis et al: "Auxin Receptors and Plant Development: A New Signaling Paradigm", Annual Review of Cell and Developmental Biology, vol. 24, no. 1,2008-11-01, pages 55-80 discloses the signalling pathway of the plant hormone auxin upon binding auxin receptors with the focus on the resulting unbiquitination and subsequent degradation events.

Regarding ABP1 which was first separated from cotyledons of a corn, there are accumulated studies on separation of a gene for the 22kD protein, binding of the protein to auxin, and physiological function, etc., and consequently it was found that ABP1 is related to the physiological effect of auxin. However, as the IAA dissociation constant of ABP1 is relatively high and the auxin signal transduction pathway after binding with auxin cannot be explained, it remains uncertain whether or not ABP1 is truly a receptor for auxin. Meanwhile, T1R1, which is identified through a series of studies on a group of genes which show different expression under the treatment with auxin hormone, was found to be a receptor for auxin hormone that is involved in the turnover of the protein degradation. However, except the regulation of the expression of auxin gene, not all of the various controlling activities of auxin hormone, for example the physiological activity of auxin including acidification of a cell wall by auxin and bell-like dose response of auxin, etc., can be explained by T1R1, and it remains as a problem.

According to the previous studies, inventors of the present invention identified the water-soluble protein from young seedlings and roots of a rice plant, which is directly related to the activation of a proton pump in plant plasma membrane by auxin (Kim et al. (1997) FEBS Lett 409, 273-276*;* Kim et al. (1998) FEBS Lett 438, 241-244*;* Kim et al. (2000) Plant Growth Regul 32, 143-150; Kim et al. (2001) J Biol Chem 276, 10730-10736). The size of the protein determined by the inventors was 57kD, and based on the characteristics that are found in an auxin receptor like activation of a proton pump by a protein-protein interaction, IAA binding constant, and the biochemical property of directly mediating bell-like IAA-dose response which affects proton pump activation and also in view of the size of the protein, it was named "ABP57."

### DETAILED DESCRIPTION OF THE INVENTION

### Problems to be Solved by the Invention

The present invention is devised in view of the needs described above. The inventors of the present invention newly isolated the gene of auxin receptor ABP57 that is related to activation of a proton pump in a plasma membrane of a plant, and found out that the gene can be used for improvement of traits that are useful in agriculture like growth and development of a plant, etc., and therefore completed the invention.

### Technical Means for Solving the Problems

In order to solve the problems descried above, the present invention provides a method of improving traits of a plant comprising transforming a plant with a recombinant plant expression vector, wherein the plant expression vector comprises a gene encoding an auxin receptor protein involved in the activation of a proton pump in the plasma membrane of a plant derived from rice, wherein the protein has an amino acid sequence of SEQ ID NO:1, wherein the improved traits are increased plant growth or increased seed size.

Furthermore, described is a plant having improved traits by transformation with a recombinant plant expression vector, wherein the plant expression vector comprises a gene encoding an auxin receptor protein involved in the activation of a proton pump in the plasma membrane of a plant derived from rice, wherein the protein has an amino acid sequence of SEQ ID NO:1 as well as seeds thereof.

Furthermore, the invention relates to the use of a recombinant plant expression vector, wherein the plant expression vector comprises a gene encoding an auxin receptor protein involved in the activation of a proton pump in the plasma membrane of a plant derived from rice, wherein the protein has an amino acid sequence of SEQ ID NO:1 for producing by transformation a plant having increased plant growth or a seed having increased seed size.

Furthermore, the present invention provides the use of a composition for improving the traits of a plant comprising a gene encoding an auxin receptor protein involved in the activation of a proton pump in the plasma membrane of a plant derived from rice, wherein the protein has an amino acid sequence of SEQ ID NO:1, wherein the improved traits are increased plant growth or increased seed size.

### Advantageous effect of the invention

According to the present invention, gene for auxin receptor ABP57 was newly identified, and according to an *in vitro* test and an *in vivo* test by which a transformant was tested in a plant, it is suggested for the first time that ABP57 gene has an ability of activating a proton pump in a plant plasma membrane and binding to auxin hormone IAA, and it is involved in plant growth which is related to the physiological function of auxin. Considering various physiological and biochemical functions of a proton pump in plant plasma membrane, with the novel gene identified by the present invention, various industrial applications like development of an agent for promoting growth of a crop plant or an agent for protecting a crop plant, etc. are expected.

### BRIEF DESCRIPTION OF THE DRAWINGS

Fig.1 shows the SDS-PAGE electrophoresis of purified auxin receptor protein ABP57 that is derived from young seedlings of a rice plant and the results of amino acid sequencing of the peptide fragment that is obtained from trypsin digestion.
Fig. 2 shows the ORF amino acid sequence of ABP57 gene which is obtained by BLASTP analysis using the NCBI's protein database and the amino acid sequence information of the peptide fragment.
Fig. 3 shows the electrophoresis of the total RNA derived from rice seedlings (lane 1) and gene fragment of ABP57 that is amplified from genomic DNA (lane 2), together with the characteristics of the presumed protein that is encoded by a group of genes that are analogous to ABP57, as obtained by BLASTN analysis using full length cDNA database of a rice plant (KOME).
Fig. 4 shows the result of the comparison of amino acid sequence of the proteins that are encoded by a group of genes analogous to ABP57.
Fig. 5 shows the result of expression analysis of a group of genes analogous to ABP57 that are obtained from callus, stem and roots of rice based on microarray analysis data.
Fig. 6 shows a process of purifying ABP57 based on production of MBP (maltose binding protein)-fused ABP using E. coli system and a column.
Fig. 7 shows the assay result of proton pump in plasma membrane of a rice plant by using recombinant ABP57.
Fig. 8 shows the assay result of the interaction between the proton pump and ABP57 based on Hill Plot analysis in which rice plasma membrane activation was determined in terms of the dependency on ABP57 concentration. rABP57: recombinant ABP57 protein; nABP57: natural ABP57 protein.
Fig. 9 shows the assay result of the ABP57's binding property for IAA (indole-3-acetic acid) based on Scatchard plot analysis. rABP57: recombinant ABP57 protein; nABP57: natural ABP57 protein.
Fig. 10 shows a schematic diagram of a vector for transforming *Arabidopsis thaliana* with ABP57 gene and the result of gene expression analysis for the transformant.
Fig.11 shows a photograph (A) and a graph (B) in which plant growth properties are compared between the *Arabidopsis thaliana* transformed with ABP57 and the control group.
Fig.12 shows a photograph in which germination properties are compared between the rice plant (over-expression strain) transformed with ABP57 gene and the control group (Nakdong variety).

### MODES FOR CARRYING OUT THE INVENTION

In order to achieve the object of the invention described above, the present invention discloses the auxin receptor protein which is involved in the activation of a proton pump (H⁺-ATPase) in plasma membrane of a plant derived from rice, wherein the protein has the amino acid sequence of SEQ ID NO: 1. The amino acid sequence of SEQ ID NO: 1 to 5 has Protein Id of BAC10824 (ABP57), BAC10825, BAC79737, BAC79739 and BAC83785, respectively. The auxin receptor protein is ABP57 protein consisting of the amino acid sequence of SEQ ID NO: 1.

The auxin receptor protein is a protein having an amino acid sequence of SEQ ID NO: 1 that is isolated from rice (*Oryza sativa*). Further disclosed are functional equivalents of said proteins. The term "functional equivalent" means that, as a result of addition, substitution or deletion of amino acid residues, it has an amino acid sequence with at least 70%, preferably at least 80%, more preferably at least 90%, still more preferably at least 95% homology with the amino acid sequence that of SEQ ID NO: 1, thus indicating a protein which has substantially the same physiological activity as the protein of SEQ ID NO: 1.

Further, the present invention discloses a gene which encodes the auxin receptor protein. The gene disclosed herein includes both genomic DNA and cDNA which encode the auxin receptor protein. The gene disclosed herein may comprise a nucleotide sequence of SEQ ID NO: 6 to 10.
The nucleotide sequence of SEQ ID NO: 6 to 10 has a cDNA Id of AK072975, AK073714, AK102706,
AK102508 and AK103212, respectively. Preferably, the gene is AK072975 consisting of the nucleotide sequence of SEQ ID NO: 6. Variants of said nucleotide sequence are also disclosed.

Specifically, said gene may comprise a nucleotide sequence with at least 70%, preferably at least 80%, more preferably at least 90%, still more preferably at least 95% homology with the nucleotide sequences of SEQ ID NO: 6. The "sequence homology %" for a certain polynucleotide is determined by comparing two nucleotide sequences that are optimally arranged with a region to be compared. In this regard, a part of the polynucleotide sequence in a region to be compared may comprise an addition or a deletion (i.e., a gap) compared to a reference sequence (without any addition or deletion) relative to the optimized arrangement of the two sequences.

The inventors of the present invention purified the auxin receptor ABP57 from the stems of rice seedling, and by digesting the purified protein with trypsin and analyzing it with ESI-MS/MS, amino acid information was obtained for the four peptides. Using non-redundant protein sequence database of NCBI, BLAST search was carried out for the amino acid sequence of the four peptides. As a result, a gene (AK072975) of which function has not been known yet was identified. With the registered nucleotide sequence of the gene, PCR primer was designed, and total RNA and genomic DNA from rice seedlings were amplified to obtain a gene fragment. As a result of the nucleotide sequence analysis, it shows 100% match with AK072975. ORF of the gene consists of 1,539 bp and encodes the protein of 58.7kDa (pI 7.97) which consists of 512 amino acids.

By using the information of the nucleotide sequence of a gene above, full-length cDNA database search with KOME (Knowledge-based Oryza Molecular Biological Encyclopedia) and EST database search with Maize Genome Sequence Consortium were carried out. As a result, in addition to AK072975, four kinds of analogous genes (AK073714, AK102508, AK102706 and AK103212) with unknown function were identified from a rice plant, and ORF of each gene shows 53 to 73% of amino acid homology, respectively. Further, one gene with sequence homology to maize and unknown function was also identified. As a result of rice 60k microarray analysis, AK072975 and the analogous genes were found to be expressed in callus, stem and roots of a rice plant.

In order to confirm whether or not the protein encoded by the gene described above has an ability of activating a proton pump, a protein expression vector comprising the isolated gene was constructed, expressed in E. coli, and then the recombinant protein was purified. The recombinant protein was tested as a sample to determine an ability of activating a proton pump in rice plasma membrane and an ability of binding to auxin. As a result, it was found that the recombinant protein has all the characteristics, and therefore identified as a gene which encodes ABP57.

Further, disclosed is a recombinant vector comprising the gene encoding the auxin receptor protein of the invention. The recombinant vector is a recombinant plant expression vector.

The term "recombinant" indicates a cell which replicates a heterogeneous nucleotide or expresses said nucleotide, a peptide, a heterogeneous peptide, or a protein encoded by a heterogeneous nucleotide. Recombinant cell can express a gene or a gene fragment in a form of a sense or antisense, that are not found in natural state of cell. In addition, a recombinant cell can express a gene that is found in natural state, provided that said gene is modified and re-introduced into the cell by an artificial means

The term "vector" is used herein to refer DNA fragment (s) and nucleotide molecules that are delivered to a cell. Vector can replicate DNA and be independently reproduced in a host cell. The terms "delivery system" and "vector" are often interchangeably used. The term "expression vector" means a recombinant DNA molecule comprising a desired coding sequence and other appropriate nucleotide sequences that are essential for the expression of the operatively-linked coding sequence in a specific host organism. The promoter, enhancer, termination signal and polymerization signal which can be used by eukaryotic cells are well known in the art.

A preferred example of plant expression vector is Ti-plasmid vector which can transfer a part of itself, i.e., so called T-region, to a plant cell when the vector is present in an appropriate host such as *Agrobacterium tumefaciens.* Other types of Ti-plasmid vector (see, EP 0 116 718 B1) are currently used for transferring a hybrid gene to protoplasts that can produce a new plant by appropriately inserting a plant cell or hybrid DNA to a genome of a plant. Especially preferred form of Ti-plasmid vector is a so-called binary vector which has been disclosed in EP 0 120 516 B1 and USP No. 4,940,838. Other vector that can be used for introducing the DNA of the present invention to a host plant can be selected from a double-stranded plant virus (e.g., CaMV), a single-stranded plant virus, and a viral vector which can be originated from Gemini virus, etc., for example a non-complete plant viral vector. Use of said vector can be advantageous especially when a plant host cannot be appropriately transformed.

Expression vector may comprise at least one selective marker. Said selective marker is a nucleotide sequence having a property that can make a target gene get selected by a common chemical method. Example includes, a gene resistant to herbicide such as glyphosate and phosphinotricine, and a gene resistant to antibiotics such as kanamycin, G418, bleomycin, hygromycin, and chloramphenicol, but not limited thereto.

According to the expression vector disclosed herein, the promoter can be CaMV 35S promoter, actin promoter, ubiquitin promoter, pEMU promoter, MAS promoter, or histone promoter, but not limited thereto. The term "promoter" indicates a region of DNA located upstream of a structure gene, and it corresponds to a DNA molecule to which an RNA polymerase binds to initiate transcription. The term "plant promoter" indicates the promoter that can initiate transcription in a plant cell. The term "constitutive promoter" indicates the promoter that is active under most environmental conditions and cell growth or differentiation state. Since selection of a transformant can be made for various tissues at various stages, the constitutive promoter may be preferred for the present invention. Thus, selection property is not limited by a constitutive promoter.

In the above-described recombinant vector, any kind of a typical terminator can be used. Example includes, nopalin synthase (NOS), rice α-amylase RAmyl A terminator, phaseoline terminator, and a terminator for Octopine gene of *Agrobacterium tumefaciens,* etc., but are not limited thereto. Regarding the necessity of terminator, it is generally known that such region can increase a reliability and an efficiency of transcription in plant cells. Therefore, the use of terminator is highly preferable in view of the context of the present invention.

Further disclosed is a host cell that is transformed with the recombinant vector disclosed above. Any kind of a host cell known in the pertinent art can be used if stable and continuous cloning and expression of the vector disclosed above can be achieved by using it. Examples include strains belonging to the genus *Bascillus* such as *E. coli* JM109, *E*. *coli* BL21, *E. coli* RR1, *E*. *coli* LE392, *E. coli* B, *E. coli* X 1776, *E*. *coli* W3110, *Bascillus subtilus, Bascillus thuringiensis,* and the like, *Salmonella typhimurium,* intestinal flora and strains such as *Serratia marcescens* and various *Pseudomonas Spp.* and the like.

In addition, when the vector disclosed above is transformed in an eukaryotic cell, a host cell such as *Saccharomyce cerevisiae,* an insect cell, a human cell (e.g., CHO cell line (Chinese hamster ovary), W138, BHK, COS-7, 293, HepG2, 3T3, RIN and MDCK cell line), a plant cell line and the like can be used. According to the present invention, the host cell is a plant cell.

When a host cell is a prokaryotic cell, transfer of the vector disclosed above into a host cell can be carried out according to CaCl₂ method, Hanahan's method (Hanahan, D., J. Mol. Biol., 166:557-580 (1983)), and an electroporation method, etc. In addition, when a host cell is an eukaryotic cell, the vector disclosed above can be transferred into a host cell according to a microscopic injection method, calcium phosphate precipitation method, an electroporation method, a liposome-mediated transformation, DEAE-dextran treatment method and a gene bombardment method, etc. Further, the present invention provides a method of improving traits of a plant comprising transforming a plant with a recombinant plant expression vector, wherein the plant expression vector comprises a gene encoding an auxin receptor protein involved in the activation of a proton pump in the plasma membrane of a plant derived from rice, wherein the protein has an amino acid sequence of SEQ ID NO:1, wherein the improved traits are increased plant growth or increased seed size.

Determination of a physiological activity of a gene using an *in vivo* system is very important. In this connection, the inventors of the present invention over-expressed the ABP57 gene in *Arabidopsis thaliana* as a model plant, and observed any characteristics of the growth. As a result, it was found that the growth was at least two times faster than the control plant. Considering the *in vitro* test relating to the activation of a proton pump, it is believed that the growth promotion by ABP57 gene as expressed in *Arabidopsis thaliana* is closely related to the activation of a proton pump present in the plasma membrane of *Arabidopsis thaliana.*

Further, disclosed is a plant having improved traits by transformation with a recombinant plant expression vector, wherein the plant expression vector comprises a gene encoding an auxin receptor protein involved in the activation of a proton pump in the plasma membrane of a plant derived from rice, wherein the protein has an amino acid sequence of SEQ ID NO: 1. The plant is preferably a plant with promoted growth. The plant includes monocot and dicot plants. Examples of monocot plant include rice, wheat, barley, bamboo shoot, corn, taro, asparagus, onion, garlic, scallion, leek, wild rocambole, hemp, and ginger, but not limited thereto. Examples of dicot plant include, tobacco, *Arabidopsis thaliana,* eggplant, pepper, tomato, potato, burdock, crown daisy, lettuce, Chinese bellflower, chard, spinach, sweet potato, celery, carrot, coriander, parsley, Chinese cabbage, cabbage, leaf mustard, radish, watermelon, melon, cucumber, zucchini, gourd, strawberry, soy bean, mung bean, kidney bean, sweet pea and the like, but not limited thereto. The plant is preferably *Arabidopsis thaliana* or rice.

Further, described is a seed obtained from the plant having improved traits by the transformation with the recombinant plant expression vector disclosed above. Still further, the present invention provides the use of a composition for improving the traits of a
plant comprising a gene encoding an auxin receptor protein involved in the activation of a proton pump in the plasma membrane of a plant derived from rice, wherein the protein has an amino acid sequence of SEQ ID NO:1, wherein the improved traits are increased plant growth or increased seed size.

It is preferable that the gene consists of the nucleotide sequence of SEQ ID NO: 6. Preferably, the improvement of traits of a plant is a promotion of plant growth.

The present invention will now be described in greater detail with reference to the following examples.

### EXAMPLES

### Example 1: Amplification and nucleotide sequence determination of ABP57 gene by RT-PCR method

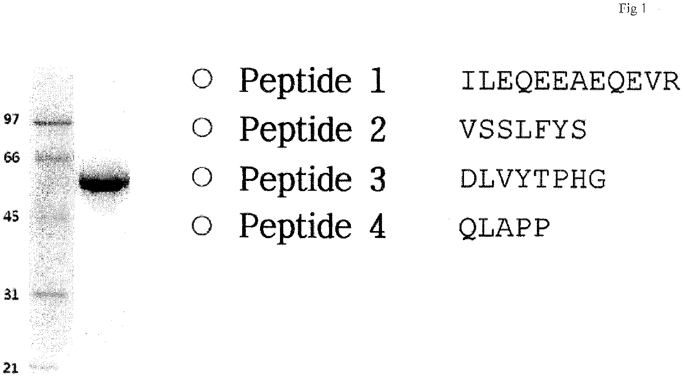

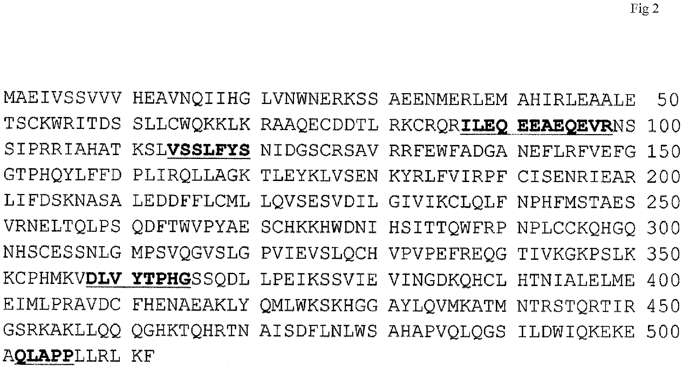

By using DEAE, CM ion exchange chromatography and tryptophan affinity chromatography, ABP567 was purified from the extract of rice seedlings of Nakdong variety. The protein was digested with trypsin, and the amino acid sequence of the digested protein was analyzed by ESI-MS/MS. As a result, the amino acid sequence information of four peptides was obtained (Fig. 1). As a result of the search of the amino acid sequence information of the peptide against NCBI's protein sequence database, one gene comprising all four peptides was found (AK072975), and the PCR primers were prepared based on the nucleotide sequence information of the gene (Fig. 2).
Forward primer (ABP57-5'): 5'-ATGGCAGAGATTGTTAGTTC-3' (SEQ ID NO: 11)
Reverse primer (RAPK1-2): 5'-CTAAAATTTCAGGCGCAGTA-3' (SEQ ID NO: 12)

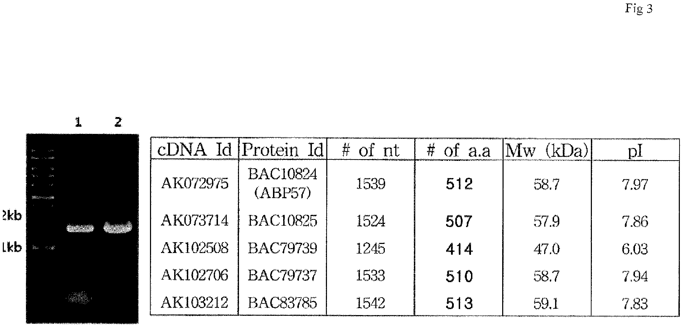

From the stems of rice seedlings of Nakdong variety, which have been grown for 4 weeks to isolate the gene, RNA and genomic DNA were isolated and amplified by RT-PCR. The amplified cDNA fragment (1.5 kb) was cloned into pGEM T-easy vector (Promega), and then the entire base sequencing was carried out. The amplified ABP57 gene encodes the protein of 58.7 kDa, which consists of 512 amino acids with 1,539 bp of gene (Fig. 3), and it shows 100% sequence homology with AK072975 registered with NCBI.

### Example 2: Obtainment of nucleotide sequence information of a gene which is analogous to ABP57 and gene expression analysis

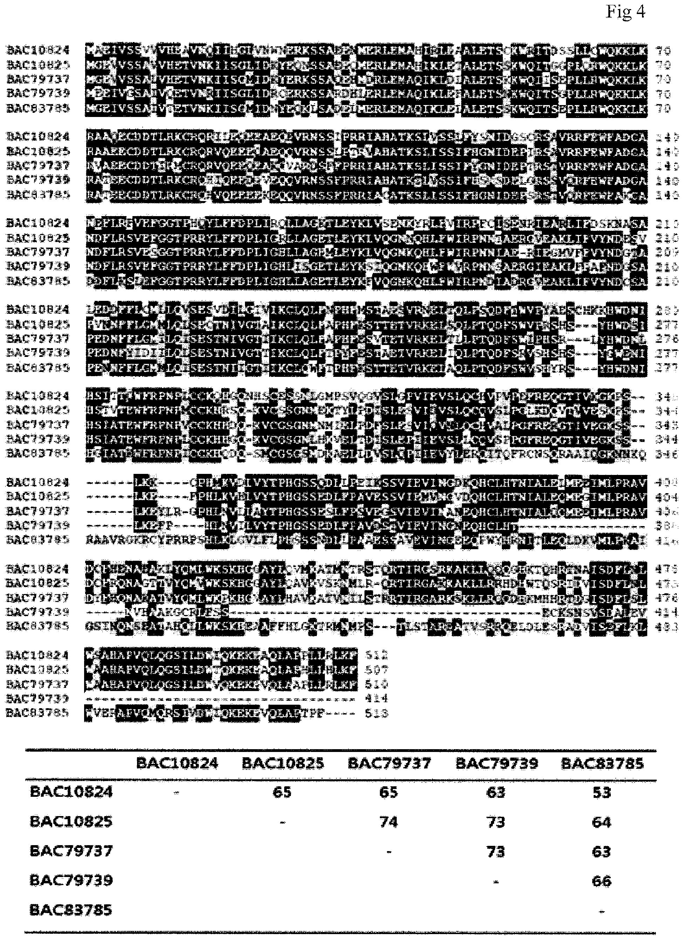

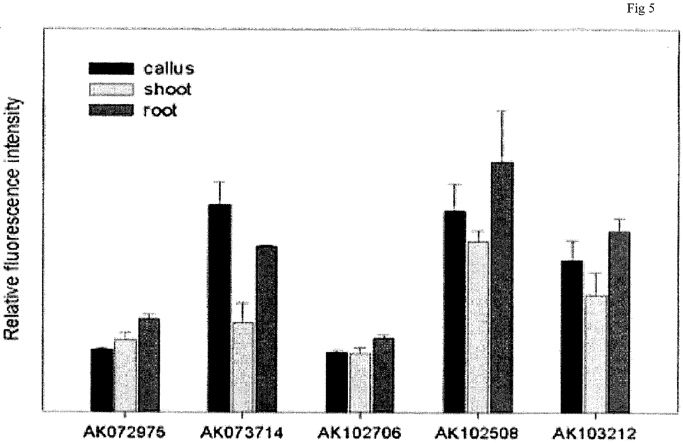

By using the nucleotide sequence information of the gene above, full-length cDNA database search with KOME (Knowledge-based Oryza Molecular Biological Encyclopedia) and EST database search with Maize Genome Sequence Consortium were carried out. As a result, in addition to AK072975, four kinds of analogous genes (AK073714, AK102508, AK102706 and AK103212) were identified from a rice plant, and ORF of each gene shows 53 to 73% of amino acid homology, respectively. Further, one gene with sequence homology to maize was also identified (Fig. 3 and Fig. 4). As a result of rice 60k microarray analysis, AK072975 and the analogous genes were found to be expressed in callus, stem and roots of a rice plant (Fig. 5).

### Example 3: Activation of a proton pump in plant plasma membrane by ABP57 recombinant protein

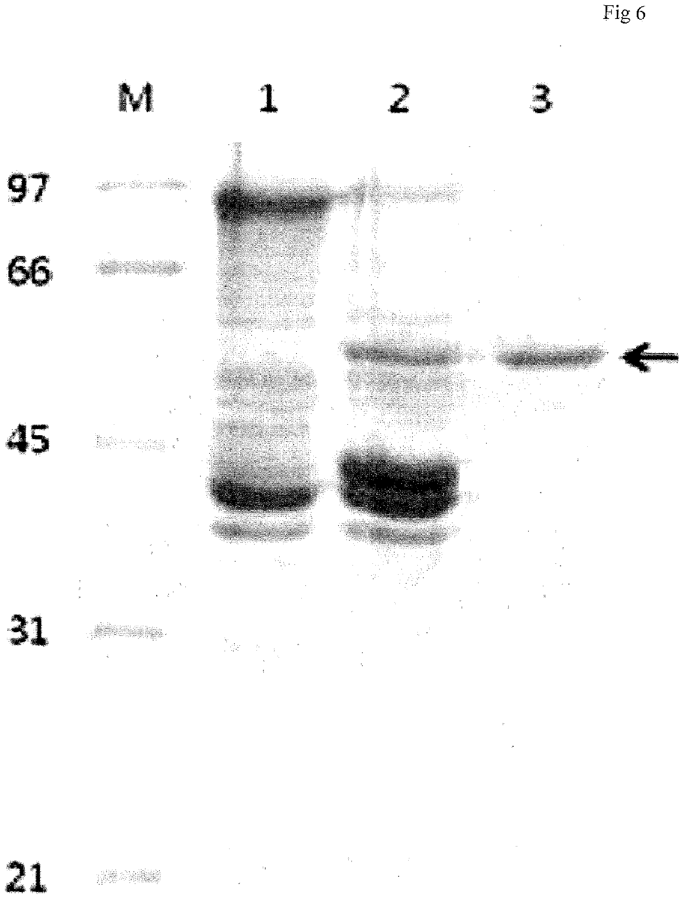

In order to produce ABP57 recombinant protein in E. coli, full-length ORF of ABP57 cDNA was cloned in BamH1 and HindIII sites of pMAL-c2x (New England Biolabs). IPTG was added to culture medium for E. coli (E. coli Rosetta(DE3)/pLysS) comprising pMAL-c2x/ABP57 vector to induce the expression of ABP57. By using an amylase resin, MBP-fused ABP57 was purified from the extract of E. coli. To the fused protein, TEV (Tobacco Etch Virus) protease was added (1%) and incubated for 12 hours at 4°C for a reaction to remove MBP (maltose binding protein) which is bound to ABP57. Finally, the protein was purified by FPLC using HiPrep^{™} Phenyl FF 16/10 column (Fig. 6).

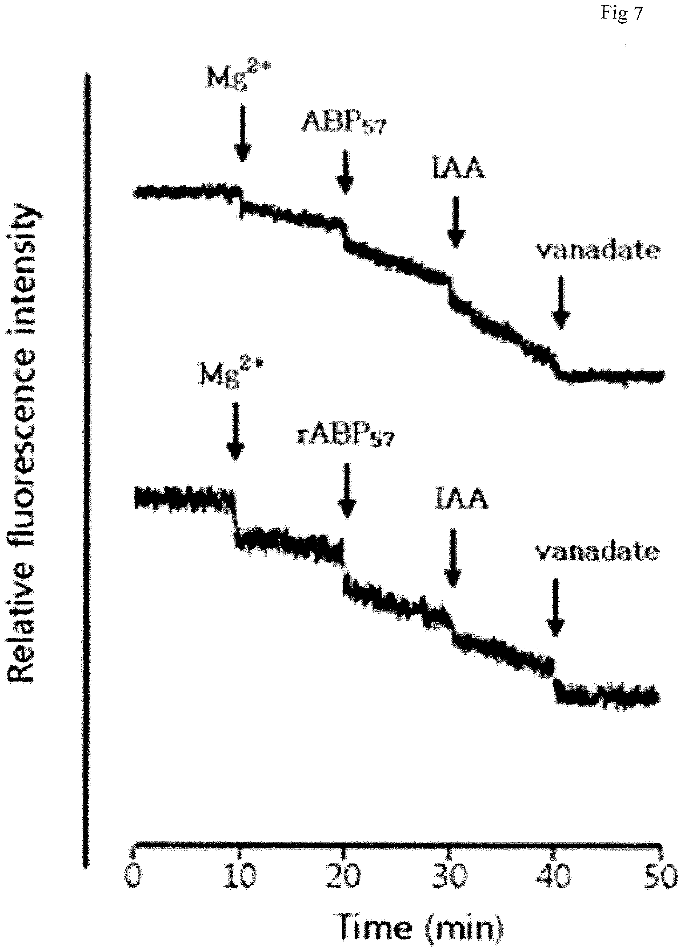

The purified protein and the proton pump in plasma membrane, which had been separated from rice, were admixed with each other, and then the activity of proton pump was measured by fluorescence analyzer. As a result, an increase in the activity of proton pump was confirmed (Fig. 7).

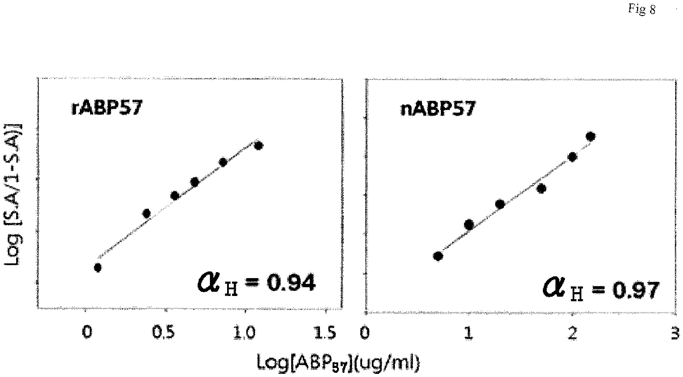

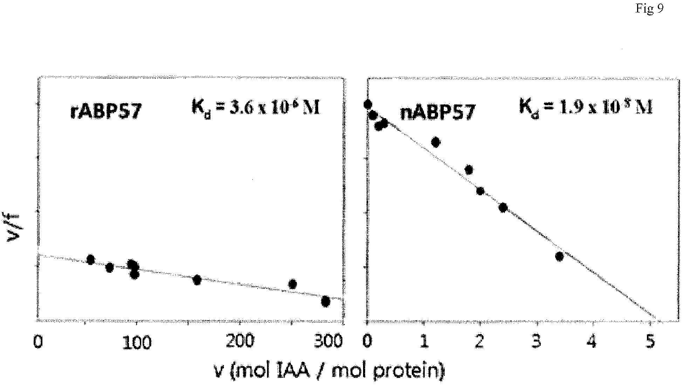

The change in degree of activating proton pump according to concentration of the recombinant protein was measured and analyzed by Hill plot analysis. As a result, the Hill coefficient was found to be 0.94, which is close to the constant 1 indicating the non-cooperative interaction between a protein and a ligand. Further, this value was very close to the Hill coefficient of 0.97, which was obtained by previous studies for the interaction between the proton pump and natural ABP57 (Fig. 8). As a result of Scatchard analysis for determining binding constant between the recombinant protein and auxin hormone IAA, the IAA dissociation constant of the recombinant protein was found to be 3.6 x 10⁻⁶ M, and the number of binding site was 1, corresponding to the low affinity binding site of natural ABP57 (Fig. 9).

### Example 4: Determination of the function of ABP57 by using a transformed plant

In order to amplify ABP57 gene by PCR and construct a plant transformation vector, a pair of PCR primers was produced.
ABP57-5'-1: 5'-AGTCGGATCCATGGCAGAGA-3' (SEQ ID NO: 13)
ABP57-3'-1: 5'-AGTCCTCGAGCTAAAATTTC-3' (SEQ ID NO: 14)

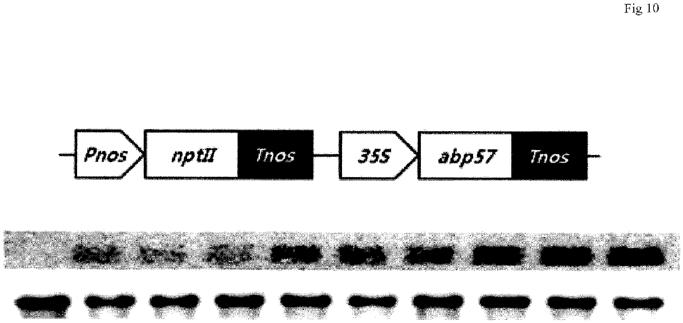

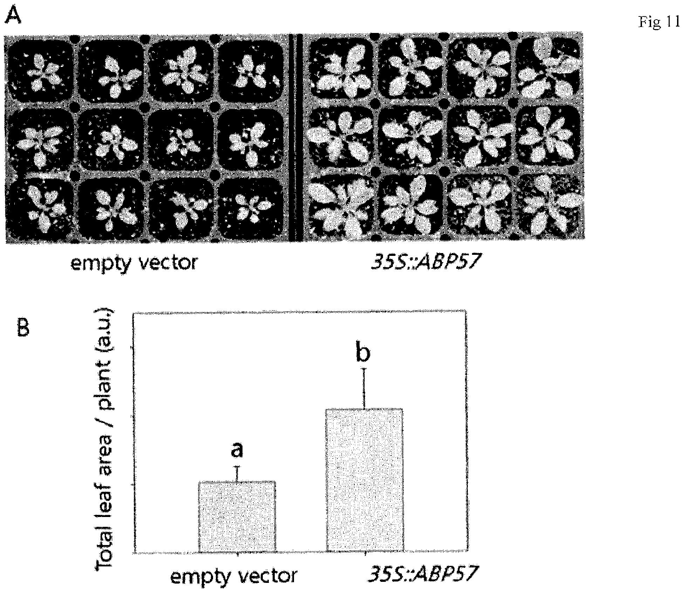

The gene fragment comprising the ORF of the amplified ABP57 was introduced to BamHI and XhoI sites in pBI111L vector (Fig. 10), and it was subsequently introduced to Agrobacterium strain C58C1. Arabidopsis Col-0 wild type host was transformed by flower dipping method. The seeds harvested therefrom were germinated in a medium comprising kanamycin to select the transformant, which was then transferred to a pot. While growing the plant, expression of the introduced gene was confirmed from the leaves by RT-PCR (Fig. 10). As a result of comparing the growth of the transformant with the control group which had been transformed only with pBI111L vector, it was found that most of the transformants showed growth that is almost two times faster than the control plant (Fig. 11).

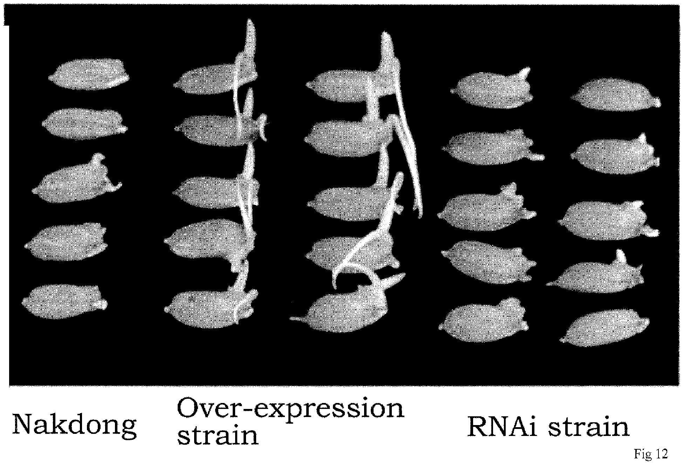

Meanwhile, the rice plant transformed with ABP57 showed promoted germination and early growth compared to the control group (Fig. 12), and the size of the seed was increased as much as 4 to 8% compared to the control group (Table 1).

**Table 1. Comparison of 100 unpolished grains between the rice transformed with ABP57 gene and the control group**

| Strain | In 100 grains of unpolished rice (g) | Increase ratio in 100 grains (%) |
|---|---|---|
| Nakdong (wild type) | 2.166 | 100% |
| 7-14-13 (transformant) | 2.341 | 108% |
| 2-17-14 (transformant) | 2.247 | 104% |

## Claims

1. A method of improving traits of a plant comprising transforming a plant with a recombinant plant expression vector, wherein the plant expression vector comprises a gene encoding an auxin receptor protein involved in the activation of a proton pump in the plasma membrane of a plant derived from rice, wherein the protein has an amino acid sequence of SEQ ID NO:1, wherein the improved traits are increased plant growth or increased seed size.

2. Use of a recombinant plant expression vector, wherein the plant expression vector comprises a gene encoding an auxin receptor protein involved in the activation of a proton pump in the plasma membrane of a plant derived from rice, wherein the protein has an amino acid sequence of SEQ ID NO:1 for producing by transformation a plant having increased plant growth or a seed having increased seed size.

3. Use of a composition for improving the traits of a plant comprising a gene encoding an auxin receptor protein involved in the activation of a proton pump in the plasma membrane of a plant derived from rice, wherein the protein has an amino acid sequence of SEQ ID NO:1, wherein the improved traits are increased plant growth or increased seed size.

## Patentansprüche

1. Verfahren zur Verbesserung von Eigenschaften einer Pflanze, umfassend das Transformieren einer Pflanze mit einem rekombinanten Pflanzenexpressionsvektor, bei dem der Pflanzenexpressionsvektor ein Gen umfasst, das ein Auxinrezeptorprotein codiert, das an der Aktivierung einer Protonenpumpe in der Plasmamembran einer Pflanze, die von Reis abgeleitet ist, beteiligt ist, wobei das Protein eine Aminosäuresequenz von SEQ ID NO:1 besitzt, wobei die verbesserten Eigenschaften erhöhtes Pflanzenwachstum oder erhöhte Samengröße sind.

2. Verwendung eines rekombinanten Pflanzenexpressionsvektors, bei dem der Pflanzenexpressionsvektor ein Gen umfasst, das ein Auxinrezeptorprotein codiert, das an der Aktivierung einer Protonenpumpe in der Plasmamembran einer Pflanze, die von Reis abgeleitet ist, beteiligt ist, wobei das Protein die Aminosäuresequenz SEQ ID NO:1 besitzt, zur Erzeugung einer Pflanze mit erhöhtem Pflanzenwachstum oder eines Samens mit erhöhter Samengröße durch Transformation.

3. Verwendung einer Zusammensetzung zur Verbesserung der Eigenschaften einer Pflanze, umfassend ein Gen, das ein Auxinrezeptorprotein codiert, das an der Aktivierung einer Protonenpumpe in der Plasmamembran einer Pflanze, die von Reis abgeleitet ist, beteiligt ist, wobei das Protein die Aminosäuresequenz SEQ ID NO:1 besitzt, wobei die verbesserten Eigenschaften erhöhtes Pflanzenwachstum oder vergrößerte Samengröße sind.

## Revendications

1. Procédé pour améliorer des caractéristiques d'une plante, comprenant la transformation d'une plante avec un vecteur d'expression de plante recombinante, dans lequel le vecteur d'expression de plante comprend un gène qui code une protéine du récepteur de l'auxine impliquée dans l'activation d'une pompe à protons dans la membrane plasmique d'une plante dérivée de riz, dans lequel la protéine a une séquence d'acides aminés de SEQ ID NO: 1, dans lequel les caractéristiques améliorées sont une croissance de plante augmentée ou une taille augmentée des semences.

2. Utilisation d'un vecteur d'expression de plante recombinante, où le vecteur d'expression de plante comprend un gène qui code une protéine du récepteur de l'auxine impliquée dans l'activation d'une pompe à protons dans la membrane plasmique d'une plante dérivée de riz, où la protéine a une séquence d'acides aminés de SEQ ID NO: 1, pour la production d'une plante avec une croissance de plante augmentée ou une taille augmentée des semences par transformation.

3. Utilisation d'une composition pour améliorer des caractéristiques d'une plante qui comprend un gène qui code une protéine du récepteur de l'auxine impliquée dans l'activation d'une pompe à protons dans la membrane plasmique d'une plante dérivée de riz, dans laquelle la protéine a une séquence d'acides aminés de SEQ ID NO: 1, dans laquelle les caractéristiques améliorées sont une croissance de plante augmentée ou une taille augmentée des semences.


<110> REPUBLIC OF KOREA(MANAGEMENT : RURAL DEVELOPMENT ADMINISTRATION)
<120> Genes encoding auxin receptor proteins from rice and uses thereof
<130> PCT10013
<160> 14
<170> KopatentIn 1.71
<210> 1
   <211> 512
   <212> PRT
   <213> Oryza sativa
<400> 1
   Met Ala Glu Ile Val Ser Ser Val Val Val His Glu Ala Val Asn Gln
    1               5                  10                  15
   Ile Ile His Gly Leu Val Asn Trp Asn Glu Arg Lys Ser Ser Ala Glu
               20                  25                  30
   Glu Asn Met Glu Arg Leu Glu Met Ala His Ile Arg Leu Glu Ala Ala
           35                  40                  45
   Leu Glu Thr Ser Cys Lys Trp Arg Ile Thr Asp Ser Ser Leu Leu Cys
       50                  55                  60
   Trp Gln Lys Lys Leu Lys Arg Ala Ala Gln Glu Cys Asp Asp Thr Leu
   65                  70                  75                  80
   Arg Lys Cys Arg Gln Arg Ile Leu Glu Gln Glu Glu Ala Glu Gln Glu
                   85                  90                  95
   Val Arg Asn Ser Ser Ile Pro Arg Arg Ile Ala His Ala Thr Lys Ser
               100                 105                 110
   Leu Val Ser Ser Leu Phe Tyr Ser Asn Ile Asp Gly Ser Cys Arg Ser
           115                 120                 125
   Ala Val Arg Arg Phe Glu Trp Phe Ala Asp Gly Ala Asn Glu Phe Leu
       130                 135                 140
   Arg Phe Val Glu Phe Gly Gly Thr Pro His Gln Tyr Leu Phe Phe Asp
   145                 150                 155                 160
   Pro Leu Ile Arg Gln Leu Leu Ala Gly Lys Thr Leu Glu Tyr Lys Leu
                   165                 170                 175
   Val Ser Glu Asn Lys Tyr Arg Leu Phe Val Ile Arg Pro Phe Cys Ile
               180                 185                 190
   Ser Glu Asn Arg Ile Glu Ala Arg Leu Ile Phe Asp Ser Lys Asn Ala
           195                 200                 205
   Ser Ala Leu Glu Asp Asp Phe Phe Leu Cys Met Leu Leu Gln Val Ser
       210                 215                 220
   Glu Ser Val Asp Ile Leu Gly Ile Val Ile Lys Cys Leu Gln Leu Phe
   225                 230                 235                 240
   Asn Pro His Phe Met Ser Thr Ala Glu Ser Val Arg Asn Glu Leu Thr
                   245                 250                 255
   Gln Leu Pro Ser Gln Asp Phe Thr Trp Val Pro Tyr Ala Glu Ser Cys
               260                 265                 270
   His Lys Lys His Trp Asp Asn Ile His Ser Ile Thr Thr Gln Trp Phe
           275                 280                 285
   Arg Pro Asn Pro Leu Cys Cys Lys Gln His Gly Gln Asn His Ser Cys
       290                 295                 300
   Glu Ser Ser Asn Leu Gly Met Pro Ser Val Gln Gly Val Ser Leu Gly
   305                 310                 315                 320
   Pro Val Ile Glu Val Ser Leu Gln Cys His Val Pro Val Pro Glu Phe
                   325                 330                 335
   Arg Glu Gln Gly Thr Ile Val Lys Gly Lys Pro Ser Leu Lys Lys Cys
               340                 345                 350
   Pro His Met Lys Val Asp Leu Val Tyr Thr Pro His Gly Ser Ser Gln
           355                 360                 365
   Asp Leu Leu Pro Glu Ile Lys Ser Ser Val Ile Glu Val Ile Asn Gly
       370                 375                 380
   Asp Lys Gln His Cys Leu His Thr Asn Ile Ala Leu Glu Leu Met Glu
   385                 390                 395                 400
   Glu Ile Met Leu Pro Arg Ala Val Asp Cys Phe His Glu Asn Ala Glu
                   405                 410                 415
   Ala Lys Leu Tyr Gln Met Leu Trp Lys Ser Lys His Gly Gly Ala Tyr
               420                 425                 430
   Leu Gln Val Met Lys Ala Thr Met Asn Thr Arg Ser Thr Gln Arg Thr
           435                 440                 445
   Ile Arg Gly Ser Arg Lys Ala Lys Leu Leu Gln Gln Gln Gly His Lys
       450                 455                 460
   Thr Gln His Arg Thr Asn Ala Ile Ser Asp Phe Leu Asn Leu Trp Ser
   465                 470                 475                 480
   Ala His Ala Pro Val Gln Leu Gln Gly Ser Ile Leu Asp Trp Ile Gln
                   485                 490                 495
   Lys Glu Lys Glu Ala Gln Leu Ala Pro Pro Leu Leu Arg Leu Lys Phe
               500                 505                 510
<210> 2
   <211> 507
   <212> PRT
   <213> Oryza sativa
<400> 2
   Met Gly Glu Val Val Ser Ser Ala Val Val His Glu Thr Val Asn Lys
     1               5                  10                  15
   Ile Ile Ser Gly Leu Ile Asp Lys Tyr Glu Gln Asn Ser Ser Ala Glu
                20                  25                  30
   Glu Gln Met Glu Arg Leu Glu Met Ala His Ile Lys Leu Glu Thr Ala
               35                  40                  45
   Leu Glu Thr Ser Ser Lys Trp Gln Ile Thr Gly Gly Pro Leu Gln Arg
           50                  55                  60
   Trp Gln Lys Lys Leu Lys Arg Ala Ala Glu Glu Cys Asp Asp Thr Leu
    65                  70                  75                  80
   Arg Lys Cys Arg Gln Arg Val Gln Glu Glu Glu Gln Ala Glu Gln Gln
                   85                  90                  95
   Val Arg Asn Ser Ser Leu Pro Thr Arg Val Ala His Ala Thr Lys Ser
               100                 105                 110
   Leu Ile Ser Ser Ile Phe His Gly Asn Ile Asp Glu Pro Ile Arg Ser
           115                 120                 125
   Ala Val Arg Arg Phe Glu Trp Phe Ala Asp Gly Ala Asn Asp Phe Leu
       130                 135                 140
   Arg Ser Val Glu Phe Gly Gly Thr Pro Arg Arg Tyr Leu Phe Phe Asp
   145                 150                 155                 160
   Pro Leu Ile Gly Arg Leu Leu Ala Gly Glu Thr Leu Glu Tyr Lys Leu
                   165                 170                 175
   Val Gln Gly Asn Asn Gln His Leu Phe Trp Ile Arg Pro Asn Asn Thr
               180                 185                 190
   Ala Glu Arg Gly Val Glu Ala Lys Leu Ile Phe Val Tyr Asn Asp Glu
           195                 200                 205
   Ser Val Pro Val Asn Asn Phe Phe Leu Gly Met Met Leu Gln Leu Ser
       210                 215                 220
   Glu Cys Thr Asn Ile Val Gly Thr Ala Ile Lys Cys Leu Gln Leu Phe
   225                 230                 235                 240
   Ala Pro His Phe Lys Ser Thr Thr Glu Thr Val Arg Lys Glu Leu Ser
                   245                 250                 255
   Gln Leu Pro Thr Gln Asp Phe Ser Trp Val Pro Arg Ser His Ser Tyr
               260                 265                 270
   His Trp Asp Ser Ile His Ser Thr Val Thr Glu Trp Phe Arg Pro Asn
           275                 280                 285
   Pro Met Cys Cys Lys His Arg Ser Gln Lys Val Cys Ser Ser Gly Asn
       290                 295                 300
   Met Glu Lys Thr Tyr Leu Pro Asp Ile Ser Leu Glu Ser Val Ile Asp
   305                 310                 315                 320
   Val Ser Leu Gln Cys Gln Val Ser Leu Pro Gly Leu Lys Asp Gln Val
                   325                 330                 335
   Thr Val Val Glu Ser Lys Pro Ser Leu Lys Glu Phe Pro His Leu Lys
               340                 345                 350
   Val His Leu Val Tyr Thr Pro His Gly Ser Ser Glu Asp Leu Phe Pro
           355                 360                 365
   Ala Val Glu Ser Ser Val Ile Glu Met Val Asn Gly Val Asp Gln His
   370 375 380
   Cys Leu His Thr Asn Ile Ala Leu Glu Gln Met Glu Gly Ile Met Leu
   385 390 395 400
   Pro Arg Ala Val Asp Cys Phe Arg Gln Asn Ala Gly Thr Thr Val Tyr
   405 410 415
   Gln Met Val Trp Lys Ser Lys His Gly Gly Ala Tyr Leu Gln Ala Val
   420 425 430
   Lys Val Ser Lys Asn Met Leu Arg Gln Arg Thr Ile Arg Gly Ala Lys
   435 440 445
   Lys Ala Lys Leu Leu Arg Arg His Asp His Trp Thr Gln Ser Arg Ile
   450 455 460
   Asp Val Ile Ser Asp Phe Leu Asn Leu Trp Ala Ala His Ala Pro Val
   465 470 475 480
   Gln Leu Gln Gly Ser Ile Leu Asp Trp Thr Gln Lys Glu Lys Glu Ala
   485 490 495
   Gln Leu Ala Pro His Leu Leu His Leu Lys Phe
   500 505
<210> 3
   <211> 510
   <212> PRT
   <213> Oryza sativa
<400> 3
   Met Gly Glu Val Val Ser Ser Ala Leu Val His Glu Thr Val Asn Lys
    1               5                  10                  15
   Ile Ile Ser Gly Met Ile Asp Lys Tyr Glu Arg Lys Ser Ser Ala Gln
               20                  25                  30
   Glu His Met Asp Arg Leu Glu Met Ala Gln Ile Lys Leu Asp Leu Ala
           35                  40                  45
   Leu Glu Thr Ser Lys Lys Trp Gln Ile Ile Ser Glu Pro Leu Leu Arg
       50                  55                  60
   Trp Gln Lys Lys Leu Lys Arg Val Ala Glu Glu Cys Asp Asp Thr Ile
   65                  70                  75                  80
   Arg Met Cys Arg Gln Arg Val Gln Glu Glu Gln Glu Ala Lys Gln Val
                   85                  90                  95
   Ala Arg Asp Ser Phe Phe Pro Arg Arg Ile Ala His Ala Thr Lys Ser
               100                 105                 110
   Leu Ile Ser Ser Ile Phe Tyr Gly Asn Ile Asp Glu Pro Thr Arg Ser
           115                 120                 125
   Thr Val Arg Arg Phe Glu Trp Phe Ala Asp Gly Ala Asn Asp Phe Leu
       130                 135                 140
   Arg Ser Val Glu Ser Gly Gly Thr Pro Arg Arg Tyr Leu Phe Phe Asp
   145                 150                 155                 160
   Pro Leu Ile Gly His Leu Leu Ala Gly Glu Met Leu Glu Tyr Lys Leu
                   165                 170                 175
   Val Gln Gly Asn Lys Gln His Leu Phe Trp Ile Arg Pro Asn Asn Ile
               180                 185                 190
   Ala Glu Arg Ile Glu Gly Met Val Phe Phe Val Tyr Asn Asp Gly Thr
           195                 200                 205
   Ala Pro Glu Asp Asn Phe Phe Leu Gly Met Ile Leu Gln Ile Ser Glu
       210                 215                 220
   Ser Thr Asn Ile Val Gly Thr Ile Ile Lys Cys Leu Gln Leu Phe Ala
   225                 230                 235                 240
   Pro His Phe Glu Ser Val Thr Glu Thr Val Arg Lys Glu Leu Thr Leu
                   245                 250                 255
   Leu Pro Thr Gln Asp Phe Ser Trp Ile Pro His Ser Arg Leu Tyr His
               260                 265                 270
   Trp Asp Asn Leu His Ser Ile Ala Thr Glu Trp Phe Arg Pro Asn Pro
           275                 280                 285
   Val Cys Cys Lys His His Asp Gln Lys Val Cys Gly Ser Gly Asn Met
       290                 295                 300
   Asn Met Ile Glu Leu Pro Asp Phe Ser Leu Glu Ser Val Ile Gln Val
   305                 310                 315                 320
   Asn Leu Gln Cys His Val Ala Leu Pro Gly Phe Arg Glu Arg Gly Thr
                   325                 330                 335
   Ile Val Glu Gly Lys Ser Ser Leu Lys Glu Tyr Leu Arg Gly Pro His
               340                 345                 350
   Leu Asn Val Leu Leu Ala Tyr Thr Pro His Gly Ser Ser Glu Ser Leu
           355                 360                 365
   Phe Pro Ser Val Glu Gly Ser Val Ile Glu Val Ile Asn Ala Asn Glu
       370                 375                 380
   Gln His Cys Leu His Thr Asn Ile Ala Leu Gln Gln Met Glu Glu Ile
   385                 390                 395                 400
   Met Leu Pro Arg Ala Val Asp Tyr Phe His Gln Asn Ala Lys Ala Thr
                   405                 410                 415
   Val Tyr Gln Met Leu Trp Lys Pro Lys His Gly Val Ala Tyr Leu His
               420                 425                 430
   Ala Val Lys Ala Thr Val Asn Ile Leu Ser Thr Arg Arg Thr Ile Arg
           435                 440                 445
   Gly Ala Arg Lys Ser Lys Leu Leu Arg Gln Gln Asp His Lys Met His
       450                 455                 460
   His Arg Thr Asp Gly Ile Ser Asp Phe Leu Ser Leu Trp Ala Ala His
   465                 470                 475                 480
   Ala Pro Val Gln Leu Gln Gly Ser Ile Leu Asp Trp Val Gln Lys Glu
   485 490 495
   Lys Glu Val Gln Leu Ala Ala Pro Leu Leu Arg Leu Lys Phe
   500 505 510
<210> 4
   <211> 414
   <212> PRT
   <213> Oryza sativa
<400> 4
   Met Glu Glu Ile Val Gly Ser Ala Ile Val Gln Glu Thr Val Asn Arg
    1               5                  10                  15
   Ile Ile Ser Gly Leu Ile Asp Arg Cys Glu Arg Lys Ser Ser Ala Arg
               20                  25                  30
   Asp His Leu Glu Arg Leu Glu Met Ala Gln Ile Lys Leu Glu Phe Ala
           35                  40                  45
   Leu Glu Thr Ser Asn Lys Trp Gln Ile Thr Ser Gly Pro Leu Leu Arg
       50                  55                  60
   Trp Gln Lys Lys Leu Lys Arg Ala Thr Glu Glu Cys Asp Asp Thr Leu
   65                  70                  75                  80
   Arg Lys Cys Arg Gln His Ile Gln Glu Glu Asp Glu Val Glu Gln Gln
                   85                  90                  95
   Val Arg Asn Ser Ser Phe Pro Arg Arg Ile Ala His Ala Thr Lys Thr
               100                 105                 110
   Leu Val Ser Ser Ile Phe His Ser Asn Ser Asp Glu Leu Gly Arg Ser
           115                 120                 125
   Ser Val Gln Arg Phe Glu Trp Phe Ala Asp Gly Ala Asn Asp Phe Leu
       130                 135                 140
   Arg Ser Val Glu Phe Gly Gly Thr Pro Arg Arg Tyr Leu Phe Phe Asp
   145                 150                 155                 160
   Pro Leu Ile Gly His Leu Ile Ser Gly Glu Thr Leu Glu Tyr Lys Ser
                   165                 170                 175
   Ile Gln Gly Asn Lys Gln His Trp Phe Trp Val Arg Pro Asn Asn Ser
               180                 185                 190
   Ala Glu Arg Gly Ile Glu Ala Lys Leu Phe Phe Ala Phe Asn Asp Gly
           195                 200                 205
   Ser Ala Pro Glu Asp Asn Phe Tyr Ile Asp Ile Ile Leu Gln Leu Ser
       210                 215                 220
   Glu Ser Thr Asn Ile Val Gly Thr Thr Ile Lys Cys Leu Gln Leu Phe
   225                 230                 235                 240
   Thr Pro Tyr Phe Glu Ser Thr Ala Glu Thr Val Arg Lys Glu Leu Ile
                   245                 250                 255
   Gln Leu Pro Thr Gln Asp Phe Ser Arg Val Ser His Ser His Ser Tyr
               260                 265                 270
   Gly Trp Glu Asn Ile His Ser Ile Ala Thr Glu Trp Phe Arg Pro Asn
           275                 280                 285
   Pro Leu Cys Cys Lys His His Gly Gln Lys Val Cys Gly Ser Gly Asn
       290                 295                 300
   Leu His Lys Val Glu Leu Thr Asp Ile Ser Leu Glu Pro Ile Ile Glu
   305                 310                 315                 320
   Val Ser Leu Leu Cys Gln Val Ser Pro Pro Gly Phe Arg Glu Gln Gly
                   325                 330                 335
   Thr Ile Val Glu Gly Lys Ser Ser Leu Lys Glu Phe Pro His Leu Asn
               340                 345                 350
   Val Ile Leu Val Tyr Thr Pro His Gly Ser Ser Glu Asp Leu Phe Pro
           355                 360                 365
   Ala Val Asp Ser Thr Val Ile Glu Val Ile Asn Gly Asn Glu Gln His
       370                 375                 380
   Cys Leu His Thr Asn Val His Ala Ala Lys Gly Cys Arg Leu Phe Ser
   385                 390                 395                 400
   Ser Glu Cys Lys Ser Asn Ser Val Ser Asp Ala Leu Glu Val
                   405                 410
<210> 5
   <211> 513
   <212> PRT
   <213> Oryza sativa
<400> 5
   Met Gly Glu Ile Val Ser Ser Ala Ile Val Thr Glu Thr Val Asn Lys
   1               5                   10                  15
   Ile Ile Ser Gly Met Ile Asp Asn Tyr Glu Gln Lys Leu Ser Ala Asp
               20                  25                  30
   Glu Leu Met Glu Arg Leu Glu Met Ala Gln Ile Lys Leu Glu Leu Ala
           35                  40                  45
   Leu Glu Thr Ser Ser Lys Trp Gln Ile Thr Ser Glu Pro Leu Leu Arg
       50                  55                  60
   Trp Gln Lys Lys Leu Lys Arg Ala Thr Glu Glu Cys Asp Asp Thr Leu
   65                  70                  75                  80
   Arg Lys Cys Arg Gln His Val Gln Glu Glu Glu Glu Lys Glu Gln Gln
                   85                  90                  95
   Val Arg Asn Ser Ser Phe Pro Arg Arg Ile Ala Cys Ala Thr Lys Ser
               100                 105                 110
   Leu Ile Ser Ser Ile Phe His Gly Asn Ile Asp Glu Pro Ser Arg Ser
           115                 120                 125
   Thr Val Gln Arg Phe Glu Trp Phe Ala Lys Gly Ala Asp Asp Phe Leu
       130                 135                 140
   Lys Ser Leu Glu Phe Gly Gly Thr Pro Arg Arg Tyr Leu Phe Phe Asp
   145                 150                 155                 160
   Pro Leu Ile Gly His Leu Leu Ala Gly Glu Thr Leu Glu Tyr Lys Phe
                   165                 170                 175
   Val Gln Gly Asn Lys Gln His Leu Phe Trp Ile Arg Pro Asn Asp Ile
               180                 185                 190
   Ala Asp Arg Gly Val Glu Ala Lys Leu Ile Phe Val Tyr Asn Asp Cys
           195                 200                 205
   Ser Ala Pro Glu Asn Asn Phe Phe Leu Gly Met Met Leu Gln Ile Ser
       210                 215                 220
   Glu Ser Thr Asn Ile Ile Gly Thr Ile Ile Lys Cys Leu Gln Trp Phe
   225                 230                 235                 240
   Thr Pro His Phe Lys Ser Thr Thr Glu Thr Val Arg Lys Glu Leu Ala
                   245                 250                 255
   Gln Leu Pro Thr Gln Asp Phe Ser Trp Val Ser His Tyr Arg Ser Tyr
               260                 265                 270
   His Trp Asp Asn Ile His Gly Ile Ala Thr Lys Trp Phe Arg Pro Asn
           275                 280                 285
   Pro Ile Cys Cys Lys His Gln Asp Gln Ser Met Cys Gly Ser Gly Ser
       290                 295                 300
   Met Asp Lys Ala Glu Leu Leu Asp Val Ser Leu Gln Pro Ile Ile Glu
   305                 310                 315                 320
   Val Tyr Leu Glu Arg Gln Ile Thr Gln Phe Arg Cys Asn Ser Gln Arg
                   325                 330                 335
   Ala Ala Ile Gln Gly Lys Asn Asn Lys Gln Arg Ala Ala Val Arg Gly
               340                 345                 350
   Lys Arg Cys Tyr Pro Arg Arg Pro Ser His Leu Lys Leu Gly Val Leu
           355                 360                 365
   Phe Leu Pro His Ser Ser Ser Asn Asp Leu Leu Pro Ala Ala Glu Ser
       370                 375                 380
   Ser Ala Val Glu Val Ile Asn Gly Glu Glu Gln Pro Trp Tyr His Arg
   385                 390                 395                 400
   Asn Ile Thr Leu Glu Gln Leu Asp Lys Val Met Leu Pro Lys Ala Ile
                   405                 410                 415
   Gly Ser Ile Asn Gln Asn Ser Glu Ala Thr Ala His Gln Leu Leu Trp
               420                 425                 430
   Lys Ser Lys His Glu Ala Ala Phe Phe His Leu Gly Lys Thr Arg Met
           435                 440                 445
   Asn Met Pro Ser Thr Leu Ser Thr Ala Arg Glu Ala Thr Val Ser Arg
       450                 455                 460
   Arg Gln Glu Leu Asp Leu Glu Ser Arg Ala Asp Val Ile Ser Glu Phe
   465                 470                 475                 480
   Leu Lys Leu Trp Val Glu Arg Ala Pro Val Gln Met Gln Arg Ser Ile
                   485                 490                 495
   Val Asp Trp Ile Gln Lys Glu Lys Glu Val Gln Leu Ala Pro Thr Pro
   500 505 510
   Phe
<210> 6
   <211> 1539
   <212> DNA
   <213> Oryza sativa
<400> 6
   atggcagaga ttgttagttc tgtggttgtt catgaggcag tcaaccaaat catacatggc 60
   ttggtgaact ggaatgagcg aaaatcaagc gcagaggaga acatggagag gcttgagatg 120
   gcacacatca ggttggaggc tgcacttgag acatcctgta agtggcgaat cactgattct 180
   tcattactgt gttggcagaa gaagctgaag cgtgctgctc aagagtgcga tgacacactg 240
   cgcaaatgcc ggcaacgcat cctggaacaa gaagaggcag aacaagaagt aaggaattcc 300
   tctattccta gacgaatagc ccatgccacc aagtcattag tgtcctcctt attttacagc 360
   aatatcgatg gctcatgtag atctgctgtt cgaagatttg agtggtttgc agatggagct 420
   aatgagtttt tgagatttgt ggagtttgga ggaacaccac atcagtactt gttctttgat 480
   cctcttatca ggcaacttct tgcaggcaaa acactagagt ataaattagt gtctgaaaat 540
   aagtaccggt tgtttgtcat acggcccttc tgtatttcag agaatagaat agaggctagg 600
   ctgatctttg acagtaaaaa tgctagtgca ctggaggatg attttttcct gtgtatgctg 660
   ctacaagttt cagagagtgt ggacatactt ggcattgtaa tcaagtgctt gcagctgttt 720
   aatccccatt tcatgtctac agctgaatct gttaggaatg aacttactca gctaccttca 780
   caagatttca cctgggtgcc atatgctgaa tcatgccaca aaaaacactg ggataatatt 840
   cacagcatta ctacccaatg gtttcgccct aacccattat gctgcaagca gcatggtcag 900
   aaccacagct gtgaaagtag caatctagga atgccatctg tacagggtgt ttctctagga 960
   ccagttattg aagtgagttt gcagtgccat gtcccagttc ctgagttcag agaacaggga 1020
   acaattgtca aaggcaagcc ttctcttaaa aaatgcccgc atatgaaagt tgaccttgtc 1080
   tacacacccc atggctcatc acaagaccta ctcccagaaa tcaagagttc tgtaatagag 1140
   gtaattaatg gtgataagca acattgcttg cacacaaaca ttgccttgga actaatggaa 1200
   gagatcatgc tgccaagggc agtagattgt tttcatgaga atgcagaagc gaaattgtat 1260
   cagatgcttt ggaagtctaa acatggtggt gcatatcttc aggttatgaa ggcaaccatg 1320
   aacactcgga gcacacagag aaccattcga ggatctagga aagcgaagct gttgcaacag 1380
   cagggtcata aaacacagca tcgtacaaat gcaatctctg acttcctcaa cctttggtct 1440
   gcacatgcgc ctgtccagct gcagggctca atcctcgatt ggattcagaa agagaaggaa 1500
   gcacaattag cacctcctct actgcgcctg aaattttag                    1539
<210> 7
   <211> 2413
   <212> DNA
   <213> Oryza sativa
<400> 7
   gggtgaccga gagcgctggc ggcgagcggc ggcgccgccg ccgcagccgc cgcgcgcatg 60
   gcggcacggg gaggtaggag gcggagcatg gagggggaag ctagctgcgc cccggcgcga 120
   cggagatgag gaggcgcaag cacaagcgca cccaccgact accgccgcgg cgagcgctcg 180
   agaaaacaga acaatgcagc aaggaggaga ccaagtcatt tttttgtgtc tgttggtggg 240
   tctgtgtgga ggtgggcatg ccaagccaac ataaggtgcc aaatagaaca atttcaatgc 300
   atatggaaga cagttcattt tataattgat gctgatggac cctttcgcaa aggaagaaga 360
   cttgcaatcc tgtgaaacag agtgtggcag gcaatttcac cggtctgaac atatattact 420
   caaaagttgg ttcagttaca gaatatctgg agaaatacca gagctggacg cacatttatg 480
   gtcttcaggc ttgcagtgca atctgtttac atcattattt tcaactagtt tggtgagtta 540
   ttgtctgctg ggtactcttc ggtgaacctg tgtgaccaca aattgcttcc tattgattca 600
   actcagaagg gagggaacat gggagaggta gtcagttctg ccgttgttca tgagacagtg 660
   aacaaaatca tatctggctt gattgacaag tatgagcaaa attcaagtgc ggaggagcag 720
   atggagaggt tggagatggc gcacatcaag ttggagactg cacttgagac atccagtaag 780
   tggcagatca ctggtgggcc cttgcaacgt tggcagaaga agctgaagcg tgctgctgaa 840
   gagtgtgatg atacactgcg caaatgtagg cagcgcgtcc aagaagaaga acaggctgaa 900
   caacaagtaa ggaattcctc cttacctacg cgagttgccc atgccaccaa gtcactgata 960
   tcatccatct ttcacggcaa tatcgacgag ccgattagat ctgctgttcg aagatttgaa 1020
   tggtttgctg atggagctaa tgactttctg agatccgtgg agtttggagg gacaccacgt 1080
   cgttacttgt tctttgaccc tcttatcggg cgccttcttg caggcgaaac acttgagtac 1140
   aaattagtgc aaggaaacaa tcagcatttg ttttggatac ggccaaataa caccgcagag 1200
   cgaggagtag aagctaagtt gatctttgtt tacaatgatg aaagtgtgcc tgtgaataac 1260
   ttttttctag gtatgatgct acaactttca gagtgcacaa acatagttgg tactgcaatt 1320
   aagtgcttgc agttgtttgc ccctcatttc aagtctacaa ctgaaactgt taggaaggaa 1380
   cttagtcaac tacctacaca ggacttctca tgggtgccac gttctcattc ataccattgg 1440
   gacagtattc acagtacagt tactgaatgg ttccgcccaa acccaatgtg ttgcaagcat 1500
   cgtagtcaga aggtatgtag tagtggcaac atggagaaga catatttacc agacatttct 1560
   ctagaatcag ttattgacgt gagtttgcag tgccaagttt cactgcctgg gttaaaagat 1620
   caggtcacag ttgttgagag caaaccttct ctcaaagaat ttccgcatct gaaagttcac    1680
   cttgtctata cgccccatgg ctcttcagaa gacctattcc cagcagtcga gagttctgtg    1740
   atagagatgg ttaatggtgt tgaccaacat tgcttgcaca caaacattgc cttggaacaa    1800
   atggaaggga tcatgctgcc aagggctgta gattgttttc gtcagaatgc aggaacaaca    1860
   gtgtatcaga tggtttggaa gtccaaacat ggtggtgcat atcttcaggc tgtgaaggta    1920
   tccaagaaca tgctgaggca gagaaccatt cgaggagcca agaaagcgaa gctattgcga    1980
   cgacatgatc attggacaca gagtcgcata gatgtgatct ctgatttctt aaatctttgg    2040
   gctgcgcatg cgcctgtcca gctgcagggc tcaatcctcg attggactca gaaagaaaag    2100
   gaagcacagt tagcacctca tctactacat ctgaaatttt agatcatgta atagggactt    2160
   gtcagggaaa tagggatact tcaaagaatg aaaatgtact accccgtcac aaaatataag    2220
   cacttttagc taagaatatg gataaaaatg cttatatttt gggacaaagg gagtaatatt    2280
   ctagcagggg aagtgactta gggagttatc tccaagttat taggagtgta ttgatctgaa    2340
   atgtgtgctc catatatttg ctgctgctgt ttgcgcagcg cagaacttag taaatggtga    2400
   taacttgatg ttc                                                       2413
<210> 8
   <211> 2331
   <212> DNA
   <213> Oryza sativa
<400> 8
   gcattcttga aaactgcagt ccagttatct tgtgctaggt actctctctc tctgggtgca      60
   cctgtctgac ccccaaggcc ccaagttact tcttattgat tcaacccaga aggttaggcc     120
   ttacattgaa ctttgaattt ctcacttttt tctgtatgga atcatgcatt atttcatttc     180
   actgccccct gcatgaatat ctcttatatc acaaaattga ttcaattcag aaggctggcc    240
   ttttcatatc ctacagcatt gcactttgtt agtacttatt aggaattcac tggtctgtct     300
   tttgatttag tgattgatat agtatatatt gattcttgat cagggaggga atcctacaga     360
   ttactgaaga tgggggaggt ggtcagttct gctctcgttc atgagacagt gaacaaaatc     420
   atatctggca tgattgacaa gtatgagcga aaatcaagtg cgcaggaaca catggacagg     480
   ttagagatgg cacaaatcaa gttagatctt gcacttgaga catccaagaa gtggcagatc     540
   attagtgagc cgttgctacg ttggcagaag aagctgaaac gtgttgctga agagtgtgat     600
   gacacaatac gcatgtgcag gcagcgtgtc caggaagaac aagaggccaa acaagtagca     660
   agggattcct tctttcctag gcgaattgcc catgccacca agtcactgat atcgtccatc     720
   ttttatggca atatcgacga gccgactaga tctaccgttc gaagattcga gtggtttgca     780
   gatggagcta atgactttct gagatctgtg gagtctggag ggacaccgcg tcgttacttg     840
   ttcttcgacc ctcttattgg gcaccttctt gcaggtgaaa tgctagagta caaattggtt 900
   cagggaaaca agcaacattt gttttggata cggcccaata acattgcaga gaggatagaa 960
   ggtatggtgt tcttcgttta caatgatggc actgcacctg aggataactt ttttcttggt 1020
   atgatactac agatttcaga gagtacaaac atagttggca ctataattaa gtgcttgcag 1080
   ttgtttgctc ctcatttcga gtctgtaact gaaactgtca ggaaagaact tactctgcta 1140
   cctacacaag acttctcctg gataccacat tctcgtttat accattggga caaccttcac 1200
   agtatagcta ccgaatggtt ccgcccaaac ccagtgtgtt gcaagcatca tgatcagaag 1260
   gtatgtggta gtggcaacat gaacatgata gaattaccag acttctctct agaatcagtt 1320
   attcaagtga atttgcagtg ccatgttgca ctgcctgggt tcagagaacg gggaacaatt 1380
   gtcgagggca aatcttctct caaagaatat ctgcgtggac cgcatctgaa tgttctactt 1440
   gcctatacgc ctcacggatc ttcagaaagc ctattcccat cagtcgaggg ttctgtgata 1500
   gaggtgatta atgctaatga gcagcattgc ttgcatacaa atattgcctt gcaacaaatg 1560
   gaagagatca tgttgccgag ggccgtagat tattttcatc agaatgcgaa agcgacagtg 1620
   tatcagatgc tttggaagcc taaacatggt gttgcatatc ttcatgctgt gaaggcgacc 1680
   gtgaacattc tgagcacacg gaggaccatt cgaggagcta ggaaatcaaa gctgttgcga 1740
   caacaggatc ataagatgca tcatcgcaca gatggaatct ctgacttcct cagcctttgg 1800
   gctgcgcacg cacctgtcca gcttcagggc tcaatccttg attgggttca gaaagagaag 1860
   gaagtacaat tagcagctcc tctactgcgc ttgaaatttt agatgatata gggctcgttt 1920
   agtagagctc caactcctaa atttaactcc aggagttgag tctggagtgg ggttatggag 1980
   ccgtttaaac ccagctccac ctctttggtt cattttatga gagagttcca ttcagctcca 2040
   ctcccatttt gggtggagct aaaactgttt ggctgagctc tagctctagg agaggtggag 2100
   ctagtgctag tcaggaaatc agagaaacgt gaagtacaaa tgtaatattt taactgagaa 2160
   aagtgacttg tagggagata atatctaagt cattcggagt taactgatat gaaatgtgtg 2220
   ctacataaaa atgaaaactg aaaatgtcac ctagtggtct gctctttctt gacctctgta 2280
   actgcttctc cttctaatat atccggcaga gctcctgccg tcttttgctt c 2331
<210> 9
   <211> 1977
   <212> DNA
   <213> Oryza sativa
<400> 9
   gagcgaaggc tgactggctg agtagagctc gagcatgact gtactggtgg agcttgagct    60
   ctatctctac taaaaccaac tagccaggta tgtgaatctc aatctgaagg tagagagagt   120
   gaggaagcag agctggaaga agaacactag gtcctaagtg taacattttg taaaaattac   180
   tagcccaaag tgaaaataat agcttgttcc tagtccaagc tgcgaccatg ggaaggatta 240
   ctgaagatgg aagagatagt cggttctgct atcgttcaag agacggtaaa caggatcata 300
   tctgggttga ttgaccggtg tgagcgaaaa tcaagtgcac gggatcacct ggagaggctg 360
   gagatggcac aaatcaagtt ggagttcgca cttgagacat ccaataaatg gcagatcact 420
   agtggcccgt tgttacgttg gcagaagaag ctgaagcgtg ctactgaaga gtgtgatgac 480
   acactacgga agtgcaggca acatatccag gaagaagatg aggtggaaca acaagtaagg 540
   aattcttcct ttcctaggcg aattgcccat gccaccaaga cactggtatc ttccatcttt 600
   cacagcaata gtgatgagct gggtagatct tctgtccaaa gattcgagtg gtttgcagat 660
   ggagctaatg attttctaag atctgtggaa tttggaggca caccacgccg ttacttgttc 720
   tttgaccctc ttatcgggca ccttatttca ggtgaaacgt tagagtacaa atcaatacag 780
   ggaaacaagc agcattggtt ttgggtacgg cccaataact ctgcagagcg aggaatagaa 840
   gctaagttgt tctttgcttt caatgatggc agtgcacctg aggacaactt ttacattgat 900
   attattctac aactttcaga gagtacaaac atagttggta ctacaattaa gtgcttgcag 960
   ttgtttactc cttattttga gtctacagct gaaactgtta ggaaagaact tattcaacta 1020
   cctacacaag acttctccag ggtgtcacat tctcactcat acggttggga gaacattcat 1080
   agtatagcta ctgaatggtt ccgcccaaat ccactgtgtt gcaagcatca tggtcagaaa 1140
   gtatgtggta gtgggaactt gcacaaggta gaattaacag atatttctct agaacccatc 1200
   attgaagtga gtttactgtg ccaagtttca cctcctgggt tcagagaaca gggaacaatt 1260
   gttgagggta aatcttcact taaagaattt ccgcatctga atgttatcct tgtctatacg 1320
   ccccatggct cttcagaaga cctattccca gcagttgata gtactgtgat tgaggtgatt 1380
   aatggtaacg agcaacattg cttgcacaca aatgttcatg ctgccaaggg ctgtagattg 1440
   ttttcgtcag aatgcaaaag caacagcgta tcagatgctt tggaagtcta aacatggtgg 1500
   tgcatttctt caggctgtga aggcaacaat gaacatgcgg agcacacgga gaaccattcg 1560
   aggagccagg aaagcaaaga tgttgcgacg acatgatcgc cggacacaca atcacagaca 1620
   tgaggtcgct gacttcctca gcctttgggc tgtgcatgca cctgtccggc ttcagggctc 1680
   actccttgac tggattcgga aggagaagaa agtagcaggt cctctactga cattttagat 1740
   gatgtaatag ggactagaag ggaaacaggg ataattaaaa ttaaaagaat gggggagaaa 1800
   agtaacatgt agggagttgg tttctaagta cttaggagtg tattcatctg aaatgttcta 1860
   catatactgc tgttgtttgt ctcgtcagtt atgttggcaa atggtgataa catgatgttc 1920
   ttgccctaga agtctaatgt accagccttg ctcattcgat gtgtctggtt tcatttg 1977
<210> 10
   <211> 2189
   <212> DNA
   <213> Oryza sativa
<400> 10
   gggagtcgct gaccaccatg gcaaccacca ccgtgcacgc cactgacgcc atggccggac 60
   ggggcctctc cgccgccaga tggggattgc tctgccgccg aagggtccgc agcgaggccg 120
   cgccaactcg tggatcggcg gcgcaaggcg gtgccataat ggcgtttcct tcctcgatcc 180
   gatgccgctg ttgcttccac tgtgagccgc caccatacac gcgtgcttgt gcggcgaccc 240
   ggcgatgcgg agagggaaga aggccgccgc ttcctcgcgg cggccacggc gcgctagatc 300
   aagggaggca gctcttgcgg attgatcact gaagatggga gagatagtca gttctgccat 360
   tgttaccgag acagtgaaca aaatcatatc tggcatgatt gacaattatg agcaaaaatt 420
   aagtgcagac gagctgatgg agaggctgga gatggcacaa atcaagttgg agcttgcact 480
   tgagacatcc agtaagtggc agatcacgag tgagccgttg ctgcgttggc agaagaagct 540
   gaagcgtgct actgaagagt gtgatgacac actacgcaag tgcaggcagc atgtccagga 600
   agaagaggag aaggaacagc aagtaaggaa ttcctccttt cctaggcgaa ttgcctgtgc 660
   caccaaatca ctgatatcat ccatctttca cggcaatatc gacgagccaa gtagatcaac 720
   tgttcaaaga tttgagtggt ttgctaaggg agctgatgac tttctgaaat ctctggagtt 780
   tggagggaca cctcgccgtt acttgttctt tgaccctctt atcgggcacc ttcttgctgg 840
   tgaaacactt gagtacaaat ttgtgcaggg aaacaagcag catttgtttt ggatacggcc 900
   caatgacatt gcagatcgag gagtagaagc taagttgatc tttgtttaca acgattgcag 960
   tgcacctgag aataactttt tcctcggtat gatgctacaa atttcagaga gtacaaacat 1020
   aattggcact ataattaagt gcttgcaatg gtttacccct catttcaagt ctacaactga 1080
   aactgttagg aaggaacttg ctcagctccc tacacaagac ttctcctggg tgtcacatta 1140
   tcgttcatac cattgggaca acattcatgg tatagctact aagtggttcc gcccaaaccc 1200
   aatatgctgc aaacatcaag atcagagcat gtgtgggagt ggcagcatgg acaaggcaga 1260
   actgctagat gtttctcttc aaccaatcat tgaagtgtat ttggagcgtc aaatcacaca 1320
   atttagatgc aacagtcaga gggcagctat acaaggaaaa aacaataaac agagagcagc 1380
   tgtacgagga aaaagatgtt atccaagaag gccttcacat ctgaaacttg gggttctttt 1440
   tttgcctcat agctcttcaa atgaccttct gcctgcagct gagagttctg cagtagaggt 1500
   gatcaatggc gaggagcagc cttggtatca cagaaacatt accctggaac agctagacaa 1560
   ggtcatgctg ccaaaggcaa taggtagcat caatcagaat tctgaagcaa cagcacacca 1620
   gcttctctgg aagtcaaaac atgaagctgc attctttcat ttggggaaga cgagaatgaa 1680
   tatgccgagc acattgagca ctgctaggga agcaactgtg tcgagacgac aggaacttga 1740
   tctggagagc cgggcagatg tgatctctga gttcctcaag ttgtgggttg agcgtgcgcc 1800
   tgtccagatg cagcgctcaa ttgtagactg gattcagaaa gagaaggaag tgcagttagc 1860
   gccaacacca ttctagaatt ctagatcatg taagagagat cgatgagggt aacaagggga 1920
   atggatagga agttcttcat gatgagaaat gagccttgtt cagtcttaat ttctaagcca 1980
   gcaataactt ggtatgtaca atagggtttc atgaaagtga agcacagtct gttcagctca 2040
   gcatataagc aagctggtaa taacatggtg ttgaaatatt acgcaagaaa tccgttcact 2100
   ggatgggtaa tcgttctttt tatctgaatt tttcttgtct aatcttttgc catcattgaa 2160
   agtgtgatga tgatattgat atatagttt 2189
<210> 11
   <211> 20
   <212> DNA
   <213> Artificial Sequence
<220>
   <223> ABP57-5' primer
<400> 11
   atggcagaga ttgttagttc 20
<210> 12
   <211> 20
   <212> DNA
   <213> Artificial Sequence
<220>
   <223> RAPK1-2 primer
<400> 12
   ctaaaatttc aggcgcagta 20
<210> 13
   <211> 20
   <212> DNA
   <213> Artificial Sequence
<220>
   <223> ABP57-5'-1 primer
<400> 13
   agtcggatcc atggcagaga 20
<210> 14
   <211> 20
   <212> DNA
   <213> Artificial Sequence
<220>
   <223> ABP57-3'-1 primer
<400> 14
   agtcctcgag ctaaaatttc 20